Europäisches Patentamt

European Patent Office

Office européen des brevets

(19)

(11) **EP 1 158 450 A2**

(12) **EUROPEAN PATENT APPLICATION**

(43) Date of publication:
**28.11.2001 Bulletin 2001/48**

(51) Int Cl.⁷: **G06F 19/00**

(21) Application number: **01401305.6**

(22) Date of filing: **18.05.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **24.05.2000 US 206619 P**
**30.06.2000 US 608879**

(71) Applicant: **ROHM AND HAAS COMPANY**
**Philadelphia, Pennsylvania 19106-2399 (US)**

(72) Inventors:
• **Hook, John William**
  **Warminster, PA 18974 (US)**
• **Pietry, Daniel**
  **83600 Les Andrets de l'Esterel (FR)**

(74) Representative: **Cabinet Hirsch**
  **34, Rue de Bassano**
  **75008 Paris (FR)**

(54) **Computer-based product formulation**

(57)    A computer implemented method of formulating a product (e.g., a chemical product) includes receiving a product formulation script that guides the formulation of a product from a number of different components. The script can, e.g., set forth required and optional components and groups of related alternative components. To provide for such guidance, the script includes (i) a component selection option identifying sets of formulation components, (ii) at least one constraint to identify a numeric range associated with a property of one of the formulation components, and (iii) a target constraint.

The product formulation script is processed to determine a collection of formulation components that can be used to produce the product such that one or more properties of the product meet associated target constraint (s). Processing of the script also includes selecting from among optional formulation components. This selection can be performed by determining sets of formulation components to be used in formulating the product and determining a permitted range associated with the formulation components. The permitted ranges enabling the formulated product to meet the target constraint(s).

FIG. IA

FIG. 1B

**Description**

**CROSS-REFERENCE(S) TO RELATED APPLICATIONS**

This application claims the benefit of the filing date of U.S. provisional application serial number 60/206,619, which was filed on May 24, 2000 and U.S. utility application serial number 09/608,879, which was filed June 30, 2000.

**BACKGROUND OF THE INVENTION**

Chemical manufacturers, and manufacturers in other industries, rely on accurate information about characteristics of the products they produce. Accurate information is needed to determine how products perform under different environmental and usage situations and is used to guide research and product development. Marketing and informational literature may also need to accurately disclose product characteristics. To determine the characteristics of products, and to produce new products with desirable characteristics, manufacturers rely on the experience and advanced knowledge of research employees (e.g., analytic chemist). Researchers commonly rely on their knowledge and experience about existing products and product formulations in order to determine desirable modifications to existing products. In many cases, however, this may still be a trial-and-error endeavor requiring research employees to produce samples of modified versions of products and to test these samples to determine whether they are suitable for sale and mass production. Trial-and-error product development and testing may involve the production of a significant amount of unusable test products. This can be an expensive process. To reduce the cost in producing new products, or modifying existing products, automated tools that can help to predict product characteristics, or which can help to formulate products with particular characteristics, are desired.

**SUMMARY OF THE INVENTION**

In general, in one aspect, the invention features a computer implemented method of formulating a product (e.g., a chemical product). The method includes receiving a product formulation script. The product is formulated from a number of different components and the script guides the formulation of the product by setting forth, e.g., required and optional components and groups of related alternative components. To provide for such guidance, the script may include (i) a component selection option identifying sets of formulation components, (ii) at least one constraint to identify a numeric range associated with a property of one of the formulation components, and (iii) a target constraint. The product formulation script is processed to determine a collection of formulation components that can be used to produce the product such that one or more properties of the product meet associated target constraint(s). Processing of the script also includes selecting from among optional formulation components. This selection can be performed by determining sets of formulation components to be used in formulating the product and determining a permitted range associated with the formulation components. The permitted ranges enabling the formulated product to meet the target constraint(s).

Implementations may include one or more of the following features. The script may be processed using a formulation knowledge web that interrelates properties of the formulation components and target properties. The formulation component properties (which can be retrieved from a database) include properties of raw materials and properties associated with manufacturing processes. Processing the script can include determining formulation components to be included and/or excluded from a formulation. Ranges identified by constraints may include both ranges of values enabling target constraints to be met and ranges of values that would cause target constraints not to be met. These ranges may identify, e.g., the amount of an ingredient to be included in a formulation. Processing the script also can include determine which target constraint values are attainable and which are not attainable.

The method can be implemented in software using a general or special purpose computer system. The details of one or more embodiments of the invention are set forth in the accompanying drawings and the description below. Other features, objects, and advantages of the invention will be apparent from the description and drawings, and from the claims.

**DESCRIPTION OF THE FIGURES**

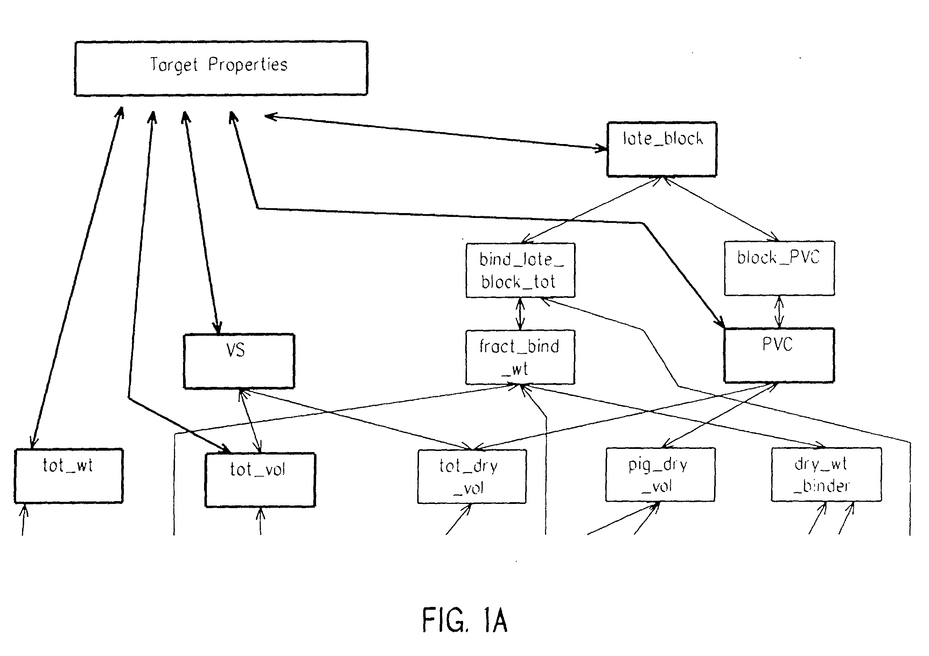

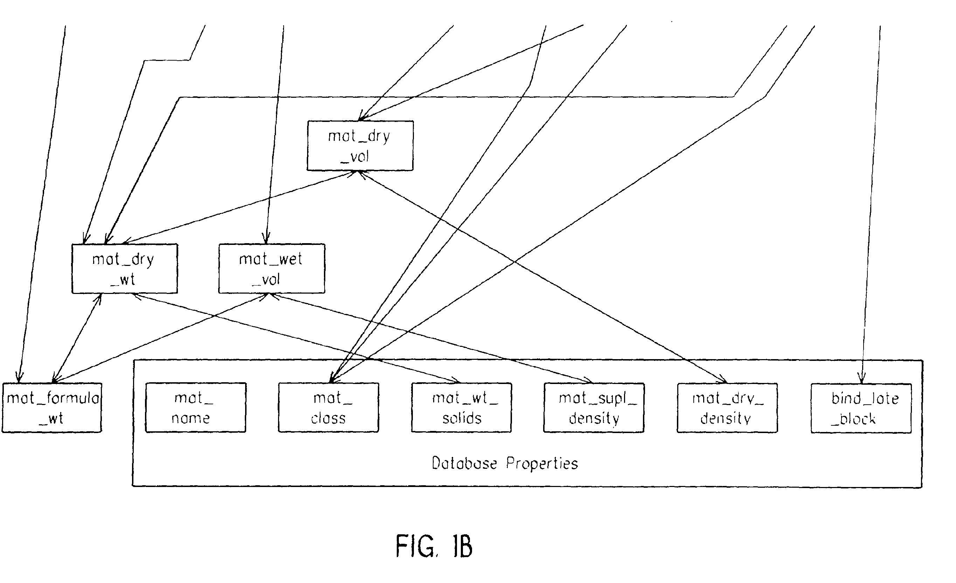

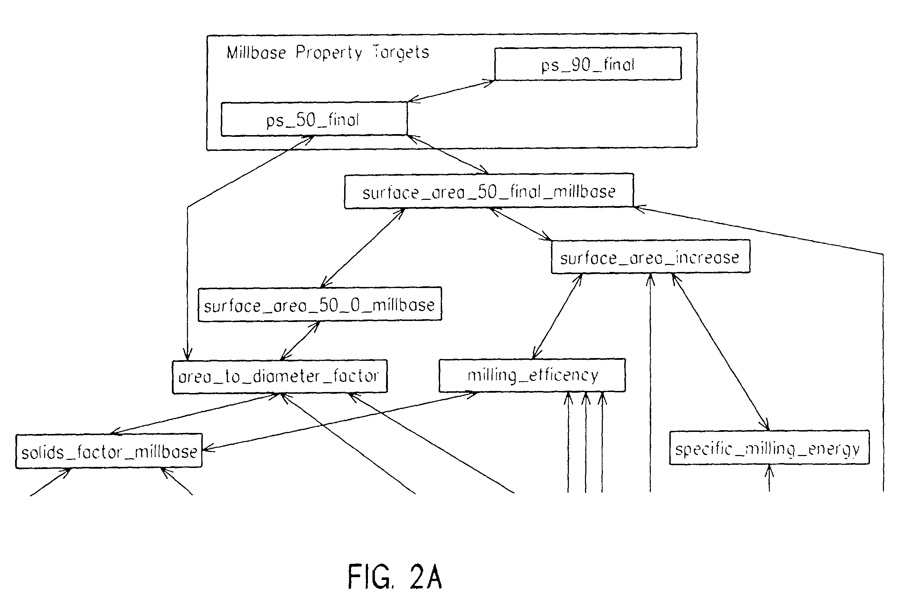

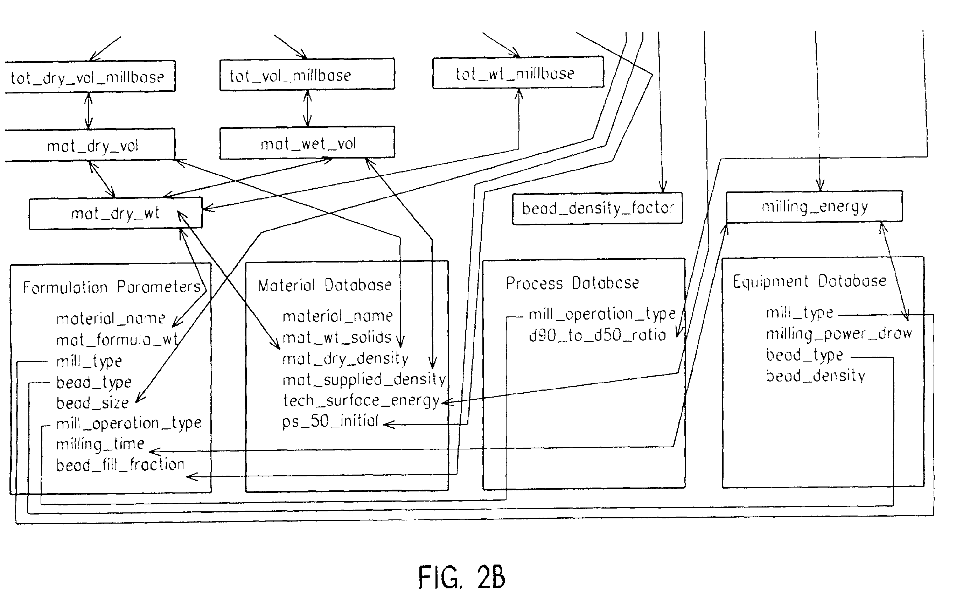

Figs. 1 and 2 are formulation knowledge representation.

**DETAILED DESCRIPTION OF THE INVENTION**

A formulation engine can help to formulate a new chemical composition (a "target composition"). This is done through the processing of static knowledge (SK) and the determination and processing of dynamic knowledge (DK).

In general, static knowledge encompasses the known, pre-determined, or use-specified values that are received by the formulation engine, while dynamic knowledge encompasses values determined, and inferences drawn, through processing by the formulation engine. Thus, dynamic knowledge is an explicit or implicit function of static knowledge and/or of other types of dynamic knowledge. Static knowledge can include, e.g., data about the intrinsic properties of various chemical compositions (e.g., mass, volume), manufacturing facilities (e.g., capacity), and manufacturing processes (e.g., temperature). Static knowledge may be represented in a database or received through user inputs. Dynamic knowledge includes similar types of information which may be undetermined prior to computation by the formulation engine. Dynamic knowledge can also include intermediate properties required for a computation. For example, if a specific processing temperature is specified by the user, then that temperature may be considered static knowledge. However, if the user indicates a range of possible values which constrains that processing temperature, then temperature is part of the dynamic knowledge, values of which may vary within the specified range during a computation. Classification of a particular variable as SK or DK is determined by the context of not only the knowledge domain, but also the specific user request.

The formulation engine can help to determine a formulation of a target composition. This determination is based on desired properties ("output constraints") of that target composition and based on specified properties of the constituent ingredients and the manufacturing processes used to produce the target composition ("input constraints"). The formulation engine can process the input constraints, static knowledge, and dynamic knowledge to determine a target formulation composition satisfying the output constraints. For example, in a paint formulation implementation, discussed below, the formulation engine can select from among a set of specified constituent ingredients, and determine amounts for those ingredients, to produce a paint having a block resistance property within a particular range.

The formulation engine determines a target composition using pre-determined and/or dynamically determined associations between static and dynamic knowledge. These associations between SK and DK are referred to as a formulation knowledge web (FKW). In general, processing using this formulation knowledge web involves (1) building the formulation knowledge web (FKW) between SK and DK, and then (2) using the formulation knowledge web (FKW) to solve a formulation problem. Building the formulation knowledge web may include both manual processes (i.e., programming steps by a programmer) and automated steps. In the example described herein, the manually determined relations are specified in the PROLOG IV language, and the automated determination and building of relations can be accomplished using inference-determining functions of the PROGLOG IV programming language.

The relationships between the SK and DK may be represented in a directional or a non-directional manner for processing by the formulation engine. If we have a dynamic knowledge set **DK** of N variables, ( $DK_n$ for n = 1 to N), and a static knowledge set **SK** of M variable ($SK_m$ for m= 1 to M), then the FKW can be represented as a series of functions:

$$DK_n = f(SK_m)$$

$$DK_{n1} = f(DK_{n2})$$

To fully calculate formulation recommendations, the formulation engine also may use the inverse functions:

$$SK_m = f^{-1}(DK_n)$$

$$DK_{n2} = f^{-1}(DK_{n1})$$

In a non-directional representation, such as the Prolog IV example below, these inverse functions are implied explicitly by the direct functions. In a less sophisticated representation, such as one might do in another language, these inverse functions could be built by the programmer or by the formulation engine.

The formulation knowledge web used in a particular implementation will differ depending on the particular implementation. For example, an implementation directed to formulating paint will have a different formulation knowledge web than an implementation directed to producing agricultural chemicals. In the discussion that follows, a generally applicable principal for building a formulation knowledge web is discussed, followed by implementation-specific examples. This general principle can be directly applied to, or adapted for, a variety of different problem domains.

The general principle of building a formulation knowledge web involves allocating variables to represent static and dynamic knowledge, and establishing relations between those variables. For example, a variable $SK_i$ can be created for each item of static knowledge. Similarly, a variable $DK_i$ can be created for each item of dynamic knowledge.

The variables $SK_i$ and $DK_i$ may be allocated by a programmer using a PROLOG IV declaration, or the variables may be dynamically determined based on inferences drawn during PROLOG IV processing. Each variable ($DK_i$) may have associated constraints that define a domain of valid settings of that variable. For example, the variable $DK_i$ may be constrained to have values between 1 and 10. Relations between variables also may be defined by a set of constraints (e.g. numerical, Boolean).

Once the FKW has been constructed, the Formulation Engine can use the FKW to process the specific user request such as DK calculation (concluding all possible values for $DK_n$) or DK optimization (concluding which value of $DK_m$ is needed to optimize $DK_n$).

A simple example of relations between variables and the use of constraints is illustrated by a calculation of Formulation Total Weight (TW). Formulation total weight is the weight of a target composition and can be determined as a sum of the weights of the individual materials in the target composition. Thus, for a target composition composed of a set of n materials, TW can be calculated as follows:

$$TW = \sum_{1}^{n} MW_i$$

As a simple example, assume that TW can be formed from two individual materials $MW_1$ and $MW_2$, and that the user has specified the following constraints: $1000 <= TW <= 1050$; $MW_1 = 800$; and $100 <= MW_2 <= 300$. The formulation engine processes this information and determine a formulation of $MW_1$ and $MW_2$ satisfying their constraints and the constraints on TW. In this case, the formulation engine recognizes that, based on the input constraints, $MW_1$ must have the fixed value of 800, and, to satisfy constraints on $MW_2$ and TW, $MW_2$ can have a value between 200 and 250. Combining $MW_1$ and $MW_2$ according to these results will produce a target composition with a weight of between 1000 and 1050.

A formulation engine can determine chemical product formulation information using a database storing knowledge about characteristics of, and interrelationships between, various raw materials, product applications, manufacturing processes, and chemical product formulations. These characteristics and interrelationships may be implemented by interconnecting different variables in a knowledge "web." This "web" is referred to herein as a formulation knowledge web.

The foregoing example illustrates the use of a formulation knowledge web for selection of material quantity. Implementations may also use a formulation knowledge web to select from among different materials or process options. For example, two different materials X and Y, each with its own constraints and properties, may satisfy a constraint associated with a target composition. A formulation knowledge web may be used to determine which of X and Y will, in fact, satisfy constraints of the target composition. Thus, a formulation knowledge web can represent knowledge about material selection, as well as quantitative calculation (e.g. material quantity, property value).

Treating both material (or process) selection and quantitative calculation at the same level of the algorithm is useful in building the FKW so that the formulation engine can simultaneously consider both the selection and the quantity needed in the user request. This can be done, for example, by expressing the material or process choice in numerical terms such as its rank ($r_i$) in the list of allowed choices. The selection then becomes a numerical function of dynamic knowledge (DK) and the FKW then is fully numeric.

Problem Solving

Determining formulation properties using the formulation engine can include calculating a resulting set of domains (i.e., intervals) for each of a set of factors (expressed as variable) influencing the formulation property. These domains may be associated with variables in an interface variable group (IVG). Example IVG variables can include material names, material quantities, formulation property values, and formulation and manufacturing process values.

In some implementations, calculating intervals can be performed by enumerating all values of variables, and then calculating results for each enumeration (i.e., the result may be determined using all permutations of permitted variable values). For example, if X and Y are integer variables used to calculate Z, and $1 <= X <= 2$, and $1 <= Y <= 3$, then calculating the interval of Z requires calculating Z for six different (X,Y) combinations (1,1), (1,2), (1,3), (2,1), (2,2), and (2,3).

A more complex challenge is presented by solving the total weight example above. While enumeration of the entire range would be possible, it is better to initially use a numeric solver which takes advantage of interval arithmetic and fixed point algorithm. In such a case, a numerical solver could make the following deductions:

- $MW_1$ is fixed at 800 by the initial constraint.

- $MW_2$ is in the range [100, 300] inclusive by the initial constraint.
- From the addition definition of TW, TW is bounded by the minimum value of 900 and the upper bound of 1100,
- From the initial constraint on TW we know TW is bounded by 1000 and 1050.
- Based on the two TW bounds in the previous two bullets, the largest minimum bound on TW is 1000, the smallest maximum bound is 1050,.
- $MW_2$ is bound by the TW definition, the fact that $MW_1$ is fixed at 800, the previous conclusion that TW is in the range [1000, 1050] inclusive. So MW2 is bounded by the [200, 250] inclusive.
- No further reductions of the variable ranges are possible so the solver stops.

In this example the solver has correctly concluded the range on $MW_2$. However, in the general case, numerical solvers may not be able to conclude the actual minimum ranges, so the solver may be combined with an enumeration algorithm which explores the reduced constraint set from the solver. This may be accomplished using a classical interval partitioning algorithm. This combination of range calculation and numeric enumeration then achieves the final reduction of the ranges more efficiently than enumeration alone, since it does not have to search the entire initial constraint space, and more reliably than interval arithmetic alone. Again, it is clear that treating material or process selections as numeric variables $DK_{ri}$ in the FKW places the selection of a specific material or process element at the same level as the numeric constraints.

Modern programming languages supporting constraint logic programming and interval arithmetic, such as the Prolog IV language, can automate such interval calculations. In the disclosure that follows, a Prolog IV-based formulation engine is described herein. Implementations may use other programming languages.

Dynamic Knowledge (DK) optimization

The formulation engine can perform dynamic knowledge optimization to find a value of a numerical variable ($V_i$) that optimizes a particular formulation characteristic. An optimum value of a variable $V_i$ is affected by the variable's direct and indirect relationships to other variables (this corresponds to direct and direct relationships between DK elements). Implementations can use one or more optimization algorithms to find an optimum value for a variable. In general, an optimization algorithm that can provide a true optimal value is desirable (preferably, the selected algorithm should provide true optimization even in the presence of local optimums). The formulation engine can provide for optimization of a variable and can identify a current domain of the other variables needed to obtain this optimization.

The Branch and Bound Algorithm is a well known example of such an optimization algorithm which can find a true optimal value even in the presence of local optimums. The enumeration algorithm defines a search tree and uses the Branch and Bound strategy to find the optimum for this iteration. That optimum ($O_n$) becomes a boundary for the next iteration, so the algorithm adds a new constraint stating that $O_{n+1} > O_n$. The iteration continues until the tree is completed.

Paint Formulation Implementation:

The operation of a formulation engine adapted for formulating paint products will now be detailed. In this example, the formulation engine is used to select a paint binder that can give good late block resistance to a paint while maintaining volume (V), volume solids (VS) and pigment volume content (PVC) within desired target ranges. The paint product formulation engine determines the paint binder using a database of raw material characteristics (each of which may be expressed by a different field in a database record), and a formulation knowledge web expressing knowledge of the different factors that affect the late block resistance.

Fig. 1 shows an example formulation knowledge web that captures paint formulation knowledge. Program source code and database values for an implementation of this formulation knowledge web are shown in Table 1.

## Table 1: Prolog IV Program Code

```
/*   Database of Materials */

material(1,'Water','Water',[0,1,1,0]).
material(2,'Propylene Glycol','Solvent',[0,1.036,1.039,0]).
material(3,'Skane M-8','Biocide',[0.45,1.032,1.0345,0]).
material(4,'Tamol 731','Dispersant',[0.25,1.104,1.605,0]).
material(5,'Triton CF-10','Surfactant',[1,1.05,1.05,0]).
material(6,'Ti-Pure R-700','TiO2',[1,4,4,0]).
material(7,'ASP-170','Extender',[1,2.58,2.58,0]).
material(8,'Rhoplex HG-74D','Binder',[0.42,1.032,1.0715,7]).
material(9,'Rhoplex HG-95P','Binder',[0.465,1.0608,1.1429,9]).
```

```
material(10,'Rhoplex Multilobe
200','Binder',[0.535,1.0648,1.1215,2]).
material(11,'Texanol','Coalescent',[0,0.95,0.95,0]).
material(12,'Acrysol RM-2020','Thickener',[0.2,1.0426,1.2569,0]).
/* End of Database of Materials */

/* Relationships */

mat_name(V):-
    material(_,V,_,_).

mat_class(V):-
    material(_,_,V,_).

mat_wt_solids(V,[V,_,_,_]).

mat_supplied_density(V,[_,V,_,_]).

mat_dry_density(V,[_,_,V,_]).

mat_late_block(V,[_,_,_,V]).


/*  Builds Quantity Relations for each material */

build_material_quantity([]).
build_material_quantity([[D,[Formula_Wt,Wet_Vol,Dry_Wt,Dry_Vol|Q]
]|Data_Quant_List]) :-
%  Gets data
    mat_supplied_density(D,Supp_Dens),
    mat_dry_density(D,Dry_Dens),
    mat_wt_solids(D,Wt_Sol),
%  Sets Interval Solver Relationships
    Wet_Vol = Formula_Wt./.Supp_Dens,
    Dry_Wt = Formula_Wt.*.Wt_Sol,
    Dry_Vol = Dry_Wt./.Dry_Dens,
build_material_quantity(Data_Quant_List).


/*  Builds Total Weight Relation */

tot_wt([],0).
tot_wt([[D,Q]|Data_Quant_List],V):-
    mat_formula_wt([D,Q],FW),
    V = FW.+.V1,
    tot_wt(Data_Quant_List,V1).


/*  Builds Total Volume Relation */
```

```
tot_vol([],0).
tot_vol([[D,Q]|Data_Quant_List],V):-
    mat_wet_vol([D,Q],WV),
    V = WV.+.V1,
    tot_vol(Data_Quant_List,V1).


/*  Builds Total Dry Volume Relation */

tot_dry_vol([],0).
tot_dry_vol([[D,Q]|Data_Quant_List],V):-
    mat_dry_vol([D,Q],DV),
    V = DV.+.V1,
    tot_dry_vol(Data_Quant_List,V1).


/*  Builds Pigment Dry Volume Relation */

pig_dry_vol(Pigment_Data_Quant_List,V):-
    tot_dry_vol(Pigment_Data_Quant_List,V).


/*  Builds Total Dry Weight Relation */

tot_dry_wt([],0).
tot_dry_wt([[D,Q]|Data_Quant_List],V):-
    mat_dry_wt([D,Q],MDW),
    V = MDW.+.V1,
    tot_dry_wt(Data_Quant_List,V1).


/*  Builds Dry Weight Binder Relation */

dry_wt_binder(Binder_Data_Quant_List,V):-
    tot_dry_wt(Binder_Data_Quant_List,V).


/*  Builds Quantity Relations for Binders */

build_fract_bind_wt_quantity([],_).
build_fract_bind_wt_quantity([[D,[_,_,Dry_Wt,_,Fract_Bind_Wt|Q]]|
Binder_Data_Quant_List],DWB)  :-
    Fract_Bind_Wt = Dry_Wt./.DWB,
    build_fract_bind_wt_quantity(Binder_Data_Quant_List,DWB).


/*  Builds Volume Solids Relation */

vs(Formulation_Data_Quant_List,V)  :-
    tot_dry_vol(Formulation_Data_Quant_List,TDV),
```

```
tot_vol(Formulation_Data_Quant_List,TV),
V = TDVA./.TV.


/*  Builds Pigment Volume Content Relation */

pvc(Pigment_Data_Quant_List,Formulation_Data_Quant_List,V) :-
    pig_dry_vol(Pigment_Data_Quant_List,PDV),
    tot_dry_vol(Formulation_Data_Quant_List,TDV),
    V = PDV./.TDV.


/*  Builds block PVC Relation */

block_pvc(Pigment_Data_Quant_List,Formulation_Data_Quant_List,V)
:-
    pvc(Pigment_Data_Quant_List,Formulation_Data_Quant_List,PVC),
    V = if(bco(PVC,0,0.3),0,if(bco(PVC,0.3,0.4),(PVC.-
.0.3).*.10,1)).


/*  Builds Binder Late Block Relation */

bind_late_block_tot([],0).
bind_late_block_tot([[D,Q]|Binder_Data_Quant_List],V):-
    mat_late_block(D,LB),
    fract_bind_wt([D,Q],FBW),
    V =(LB.*.FBW).+.V1,
    bind_late_block_tot(Binder_Data_Quant_List,V1).


/*  Builds Late Block Relation */

late_block(Binder_Data_Quant_List,
Pigment_Data_Quant_List,Formulation_Data_Quant_List,V) :-
    bind_late_block_tot(Binder_Data_Quant_List,BLBT),

block_PVC(Pigment_Data_Quant_List,Formulation_Data_Quant_List,BPV
C),
    V = BLBT.+.BPVC.*.(10.-.BLBT).
```

The formulation engine can access a database of raw materials to determine inherent characteristics of the raw materials as well as uses for those materials. For example, the program code of Table 1 includes a database of twelve raw materials. This database includes the name, classification, solid fraction, supplied density, dry density and late block resistance characteristics of each material. The following table, more fully explained below, shows the content of this example material database:

Table 2

| Mat_name | Mat_Class. | mat_wt_solids | mat_supplied_density | mat_dry density | bind_late_block |
|---|---|---|---|---|---|
| Water | Water | 0 | 1 | 1 | |

Table 2 (continued)

| Mat_name | Mat_Class. | mat_wt_solids | mat_supplied_density | mat_dry density | bind_late_block |
|---|---|---|---|---|---|
| Propylene Glycol | Solvent | 0 | 1.036 | 1.039 | |
| Skane M-8 | Biocide | 0.45 | 1.032 | 1.0345 | |
| Tamol 731 | Dispersant | 0.25 | 1.104 | 1.605 | |
| Triton CF-10 | Surfactant | 1 | 1.05 | 1.05 | |
| Ti-Pure R-700 | TiO2 | 1 | 4 | 4 | |
| ASP-170 | Extender | 1 | 2.58 | 2.58 | |
| Rhoplex HG-74D | Binder | 0.42 | 1.032 | 1.0715 | 7 |
| Rhoplex HG-95P | Binder | 0.465 | 1.0608 | 1.1429 | 9 |
| Rhoplex Multilobe 200 | Binder | 0.535 | 1.0648 | 1.1215 | 2 |
| Texanol | Coalescent | 0 | 0.95 | 0.95 | |
| Acrysol RM-2020 | Thickener | 0.2 | 1.0426 | 1.2569 | |

The Table 1 source code also includes a set of six functions to access information from the materials database. These functions are shown in pseudo-code form and further explained as follows:

Functions to Access Materials Database:

mat_name(i): Accesses a unique name associated with a material.
mat_class(i): Accesses classification information about the material. The classification information may identify uses of the material. In Table 1, material classifications include "Water", "Solvent" Binder", etc.
mat_wt_solids(i): Returns the weight fraction of material (i) which is solid
mat_supplied_density(i): Returns the density (kg/li) of a material in it's supplied form (liquid or solid)
mat_dry_density(i): Returns the density (kg/liter) of the dry solids of the material in a paint film.
bind_late_block(i): Returns the late block resistance of an 18 PVC gloss paint formulation based on binder (i)

In this example, a basic unit of each material is weight. For a particular formulation of materials, the total material formulation is the material formulation weight expressed as:
mat_formula_wt(i) is the weight of the material (i) in the formulation.

The formulation engine processes a formulation knowledge web that interrelates the materials as well as knowledge about target product formulation and manufacturing processes. In the example herein, the formulation knowledge web includes a representation of the following formulation knowledge:

The dry weight of a specific material is:

$$mat\_dry\_wt(i) = mat\_formula\_wt(i) * mat\_wt\_solids(i)$$

The volume contribution of a specific material(i) in the wet paint is:

$$mat\_wet\_vol(i) = mat\_formula\_wt(i) / mat\_supplied\_density(i)$$

The volume contribution of a specific material (i) in the dry paint is:

$$mat\_dry\_vol(i) = mat\_dry\_wt(i) / mat\_dry\_density(i)$$

The total weight of the paint is the sum of the individual material weights:

$$\text{tot\_wt} = \Sigma_{\text{all materials}} \text{ mat\_formula\_wt}(i)$$

The total volume of the paint is the sum of the individual material wet volumes:

$$\text{tot\_vol} = \Sigma_{\text{all materials}} \text{ mat\_wet\_vol}(i)$$

The total dry volume of the paint is the sum of the individual dry volumes:

$$\text{tot\_dry\_vol} = \Sigma_{\text{all materials}} \text{ mat\_dry\_vol}(i)$$

The dry volume of pigment in the paint is a similar sum, applied only to the pigments:

$$\text{pig\_dry\_vol} = \Sigma_{\text{pigments}} \text{ mat\_dry\_vol}(i)$$

The dry weight of the binder in the paint is:

$$\text{dry\_wt\_binder} = \Sigma_{\text{all binders}} \text{ mat\_dry\_wt}(i)$$

The binder fraction of a specific binder (i) is:

$$\text{fract\_bind\_wt}(i) = \text{mat\_dry\_wt}(i)/ \text{ dry\_wt\_binder}$$

The Volume Solids of the formulation (VS) is:

$$\text{VS} = \text{tot\_dry\_vol}/\text{tot\_vol}$$

The Pigment Volume Content of the formulation (PVC) is:

$$\text{PVC} = \text{pig\_dry\_vol}/\text{tot\_dry\_vol}.$$

In some cases, a material's effect on the characteristics of a formulation may be represented by a series of different formulas, the particular formula to be applied depending on the concentration of the material in the formulation. For example, the association between PVC and block resistance can depend on the concentration of PVC and may be expressed by the following set of relationships:

| tot_PVC_add | block_PVC |
|---|---|
| PVC < 30% | 0 |
| 30% ≤ PVC ≤ 40% | (tot_PVC - 0.3) * 10 |
| PVC > 40% | 1 |

The binder contribution to block resistance is:

$$\text{bind\_late\_block\_tot} = \Sigma_{i=\text{binders}} ( \text{ bind\_late\_block}(i) * \text{fract\_bind\_wt}(i))$$

The target property, Late Block, is:

late_block = bind_late_block_tot + block_PVC * (10 - bind_late_block_tot).

The formulation engine may determine formula characteristics, and may help to determine a modified formulation based on a product formulation script. The product formulation script can outline a recipe (i.e., a list of materials or ingredients) that can be used to formulate a product, constraints on particular ingredients in the recipe, options for selection from among materials, and constraints on the output product that is produced. An example list of ingredients can include the following ingredients in a basic paint formulation recipe:

Table 3:

| Starting Point Formulation | |
|---|---|
| **Material** | **Weight** |
| Water | 5.00 |
| Propylene Glycol | 40.00 |
| Skane M-8 | 2.00 |
| Tamol 731 | 8.00 |
| Triton CF-10 | 8.00 |
| Ti-Pure R-700 | 200.00 |
| ASP-170 | 0.00 |
| Water | 20.00 |
| Rhoplex HG-74D | 529.00 |
| Texanol | 30.00 |
| Acrysol RM-2020 | 40.00 |
| Water | 121.00 |

The expected properties of this starting point formulation can be calculated by the formulation engine using the formulation knowledge web of Fig. 1 (as expressed by the program code of Table 1). These starting point properties are as follows:

| **Property** | **Value** |
|---|---|
| Total Weight (kg) | 1003.00 |
| Total Volume (l) | 833.95 |
| Volume Solids (%) | 32.79 |
| PVC (%) | 18.28 |
| Late Block (0 to 10) | 7.00 |

A user may wish to determine a new paint formulation by modifying the starting point formulation based on a set of user-specified constraints. The product formulation script can identify user-specified constraints such as these material choice and material level constraints.

A material choice constraint specify groups of materials that are, to some degree, interchangeable. However, specific choices within a group may have different properties. The formulation engine can determine the specific members of a group that should be selected to satisfy user-specified formulation constraints. Material classification information in the raw materials database can be used to determine suitable materials for inclusion within a group. As an example, the database of Table 2 identifies three different binders. In modifying the starting point formulation of Table 3, a user can specify that the binder ("Rhoplex HG-74D" in Table 3) may be altered by the formulation engine and may be one of the three binders(i) Rhoplex HG-74D, (ii) Rhoplex HG-95P or (iii) Rhoplex Multilobe 200. The user may also specify material level constraints; (e.g., "the weight of the binder is to be between 400 and 600 kg inclusive). For our example, the following additional constraints are specified:

Water Level Constraint:

Let the weight of the last water in the formulation be between 0 and 300 kg inclusive.

Other Materials:

Let all other material choices and levels in the starting point be fixed to their initial values given in the formulation of Table 3.

In addition to specifying constraints on the input materials, the user can specify target constraints for the formulated (output) product. In our example, these constraints are as follows:

Let the Total Weight be any value (unconstrained);

Let the Total Volume be between 833 and 835 inclusive;

Let the Volume Solids be between 32.5% and 33.0% inclusive;

Let the PVC be less than or equal to 18.5%; and

Let the Late Block be greater than or equal to 7.0.

From our starting point formulation constraints, the formulation system, using the program knowledge in Table 1, can conclude that the following two solution spaces satisfy the user-specified input and target product constraints:

Solution #1:

Solution #1 is a formulation in which the formulation engine has selected Rhoplex HG-74D as the binder. The resultant formulation "space" is as follows:

| Material | Min Weight | Max Weight |
| --- | --- | --- |
| Water | 5.00 | 5.00 |
| Propylene Glycol | 40.00 | 40.00 |
| Skane M-8 | 2.00 | 2.00 |
| Tamol 731 | 8.00 | 8.00 |
| Triton CF-10 | 8.00 | 8.00 |
| Ti-Pure R-700 | 200.00 | 200.00 |
| ASP-170 | 0.00 | 0.00 |
| Water | 20.00 | 20.00 |
| *Rhoplex HG-74D* | *522.04* | *534.35* |
| Texanol | 30.00 | 30.00 |
| Acrysol RM-2020 | 40.00 | 40.00 |
| Water | *114.86* | *128.79* |

The specific properties of a paint produced according to solution #1 will depend on the specific material levels selected and will have the following ranges:

| Property | Min Value | Max Value |
| --- | --- | --- |
| Total Weight (kg) | 989.90 | 1016.14 |
| Total Volume (l) | 833.00 | 835.00 |
| Volume Solids (%) | 32.50 | 33.00 |
| PVC (%) | 18.15 | 18.47 |
| Late Block (0 to 10) | 7.00 | 7.00 |

<u>Solution 2</u>

Solution #2 is a formulation in which the formulation engine has selected Rhoplex HG-95P as the binder. The resultant formulation space is as follows:

| Material | Min Weight | Max Weight |
|---|---|---|
| Water | 5.00 | 5.00 |
| Propylene Glycol | 40.00 | 40.00 |
| Skane M-8 | 2.00 | 2.00 |
| Tamol 731 | 8.00 | 8.00 |
| Triton CF-10 | 8.00 | 8.00 |
| Ti-Pure R-700 | 200.00 | 200.00 |
| ASP-170 | 0.00 | 0.00 |
| Water | 20.00 | 20.00 |
| *Rhoplex HG-95P* | *502.94* | *514.80* |
| Texanol | 30.00 | 30.00 |
| Acrysol RM-2020 | 40.00 | 40.00 |
| Water | *147.35* | *160.53* |

The specific properties of a paint produced according to solution #2 will depend on the specific material levels selected and will have the following ranges:

| Property | Min Value | Max Value |
|---|---|---|
| Total Weight (kg) | 1003.29 | 1028.33 |
| Total Volume (1) | 833.00 | 835.00 |
| Volume Solids (%) | 32.50 | 33.00 |
| PVC (%) | 18.15 | 18.47 |
| Late Block (0 to 10) | 9.00 | 9.00 |

<u>Rejected Choices</u>

The formulation engine also determines that binder Rhoplex Multilobe 200 cannot satisfy the specified constraint. Therefore, the formulation engine rejects Rhoplex Multilobe 200 as a binder. In the case of Rhoplex Multilobe 200, the formulation knowledge web can determine that, for the specified constraints:

$$bind\_late\_block = 2$$

$$fract\_bind\_wt = 1$$

$$block\_PVC = 0$$

This results in the conclusion that bind_late_block_tot = 2 and that late_block = 2. This is not a valid result due to the specified constraint requiring the late_block to be at least 7. Consequently, Rhoplex Multilobe 200 is rejected by the formulation engine.

As seen from the above-example, using the user-specified constraints, formulations meeting those constraints can be determined. However, characteristics of the determined formulation solutions may not span the entire space permitted by a constraint. For example, although the initial constraints indicated that between 400 and 600 kg of binder may be included, if this range was, in fact, used in the solution formulations, other constraints may be violated. To provide further formulation guidance, the formulation engine can provide revised input level constraints to ensure that a formulation solution does, in fact, meet desired constraints. In this example, the formulation engine can make the following determinations:

- The Binder should be either Rhoplex HG-74D or Rhoplex HG-95P.
- The binder weight should be between 522.04 and 534.16 kg when using Rhoplex HG-74D and 502.94 to 514.61 kg when using Rhoplex HG-95P
- The water weight should be between 116.01 and 127.48 when using Rhoplex HG-74D and 148.50 and 159.26 when using Rhoplex HG-95P.
- The Total Weight will be between 1001.49 and 1016.22
- The Volume will be between 833 and 835 (same as input constraint)
- The Volume Solids will be 32.50 and 32.97
- The PVC will be between 18.21% and 18.46% (tighter than input)
- The Late Block Resistance will be between 7 and 9 (tighter than input)

If a user subsequently constrains the Late Block Resistance further to be above 8, the formulation engine will conclude that the correct binder will be Rhoplex HG-95P and characteristics of the resultant product will have the numeric constraints expressed above. Thus, the formulation engine can be used to determine appropriate materials and material levels to meet both input and target property constraints. Depending on the complexity of problems to be solved, implementations may use larger numbers of paint performance properties and raw materials and levels.

The following code show essential details of PROLOG IV product formulation script that specifies the basic paint formulation recipe of Table 3, as well as permitted constraints and options for formulating a paint using that basic recipe.

```
ptm([8,-1,0],
/* The following inputs enter a starting point formulation */
/* as well as permitted ranges for particular formulation */
/* and permitted selections */

/* The following 7 components have a fixed level */
[[1,'Grind',17,1,eq('Water'),5,5,1],
[2,'Grind',18,1,eq('Propylene Glycol'),40,40,1],
[3,'Grind',7,1,eq('Skane M-8'),2,2,1],
[4,'Grind',4,1,eq('Tamol 731'),8,8,1],
[5,'Grind',15,1,eq('Triton CF-10'),8,8,1],
[6,'Grind',16,1,eq('Ti-Pure R-700'),200,200,1],
[7,'LetDown',17,2,eq('Water'),20,20,1],
/* The following component allows a selection from */
/* a group of three materials */
[8,'LetDown',2,1,inlist(['Rhoplex HG-74D','Rhoplex HG-
95P','Rhoplex Multilobe 200']),400,600,1],
/* The following 2 components have fixed levels */
[9,'LetDown',3,1,eq('Texanol'),30,30,1],
[10,'LetDown',14,0,eq('Acrysol RM-2020'),40,40,1],
/* the following component can vary in the range 0-300 */
[11,'LetDown',17,3,eq('Water'),0,300,1]],

/* The following commands identify the output data */
/* being requested and output constraints */
-
```

```
/* Total volume range 833-835 */
[['Total Volume',833,835,_,_],
/* Total Weight is unconstrained */
['Total Weight',_,_,_,_],
/* Total PVC may be up to 18.5 % */
['Total PVC',_,0.185,'Percent',_],
/* Volume solids can range from 32.5 - 33 percent */
['Volume Solids',0.325,0.33,'Percent',_],
/* Late block must be equal to, or greater than, 7 */
['Late Block',7,_,'10 Scale',_],
],[]).
```

Implementations may include a graphical user interlace front-end to generate the preceding input and query code.

Millbase Implementation:

In addition to selection and determination of materials and material levels, a formulation engine can be used to determine the manufacturing, application, and formulation processes to be used for a particular material. In the example that follows, an implementation of the formulation engine is used to determine characteristics of a millbase for use in herbicide preparation.

In the preparation of herbicides for use in agricultural applications one of the process steps is the preparation of a millbase. A key property of the final millbase is the particle size distribution, which largely determines other key properties of the final formulation such as viscosity profile, stability, and suspensiblity. This distribution can be represented by the 50th percentile size and the 90th percentile size. The 50th percentile size is the size for which 50% of the particles are smaller. The 90th percentile size is the size for which 90% of the particles are smaller) (these sizes are represented by the variables $ps\_50\_final$ and $ps\_90\_final$, respectively).

The final particle size of a millbase can be predicted from the power draw of the specific mill used, the density of the beads in the mill, the process method in which the mill is used, the milling time, the initial particle size distribution, and a number of other equipment, process, and material properties. A set of variables and equations representing this type of knowledge is expressed for a single active ingredient millbase below:

Equipment Database

An equipment database identifies mill equipment and associated properties. The equipment database may include the following data fields:

    $mill\_type$: unique name of the mill used to grind the millbase
    $milling\_power\_draw$: maximum power drawn by this $mill\_type$
    $bead\_type$: unique name of the type of beads used
    $bead\_density$: density of the beads used

For this example, the equipment database is populated as follows:

| mill_type | mill_power_draw (Watts) |
| --- | --- |
| Dynomill KD-15 (15 L) | 11,250 |
| Dynomill KD-50 (49 L) | 18,750 |
| Netzsch LMC 20 (20 L) | 7,500 |
| Netzsch LMC 60 (50 L) | 18.750 |

| bead_type | bead_density (g/ml) |
|---|---|
| Sand | 2.5 |
| Glass - unleaded | 2.5 |
| Flint Pebble | 2.6 |
| Glass - leaded | 3.6 |
| Zircon Oxide (YTZ) | 6.0 |
| Tungsten Carbide | 15.0 |

Process Database

A process database identifies particular milling processes and characteristics of those processes. The process database may include the following data fields:

mill_operation_type: Unique name identifying a particular method of running a mill.
d90_to_d50_ratio: Expected ratio of the 90th percentile size to the 50th percentile size for a particular method of running the mill.

For this example, the process database may be populated as follows:

| mill_operation_type | d90_to_d50_ratio |
|---|---|
| Continuous Recirculation | 4.00 |
| One Discrete Pass | 3.75 |
| Two Discrete Passes | 3.50 |
| Three Discrete Passes | 3.25 |
| Four or More Discrete Passes | 3.00 |

Material Database

A Material database includes characteristics of raw materials. The materials database may include the following data fields:

mat_name: unique name of the material in the formulation
mat_wt_solids: weight fraction of solids of the material
mat_dry_density: density of the material in the dry formulation
mat_supplied_density: density of the material in its supplied form
tech_surface_energy: surface energy of the technical active material in the millbase
ps_50_initial: particle size of the technical active material in the millbase

For this example, the materials database is populated as follows:

| mat_name | mat_wt _solids | mat_dry _density (g/ml) | mat_supplied _density (g/ml) | tech_surface _energy (J/m2) | ps_50_ initial (microns) |
|---|---|---|---|---|---|
| Goal Tech | 100% | 1.45 | 1.45 | 0.04 | 15 |
| Kelzan | 100% | 1.50 | 1.50 | | |
| Proxel GXL | 25.3% | 1.00 | 1.13 | | |
| Water | 0% | 1.00 | 1.00 | | |

Properties of a prepared millbase may be determined, in part, based on a set of initial properties associated with various manufacturing-related variables. These variables may include the following:

| material_name | mat_formula_wt | mill_type | bead_type |
|---|---|---|---|
| bead_size | mill_operation_type | milling_time | bead_fill_fraction |

These variables can be used to calculate a set of intermediate properties of the millbase. The intermediate properties, and the calculations to obtain these properties, can include the following:

$$\text{mat\_dry\_wt(i)} = \text{mat\_formula\_wt(i)} * \text{mat\_wt\_solids(i)}$$

$$\text{mat\_dry\_vol(i)} = \text{mat\_dry\_wt(i)} / \text{mat\_dry\_density(i)}$$

$$\text{mat\_wet\_vol(i)} = \text{mat\_formula\_wt(i)} / \text{mat\_supplied\_density(i)}$$

$$\text{tot\_dry\_vol\_millbase} = \Sigma_{\text{all materials in millbase}} \text{mat\_dry\_vol(i)}$$

$$\text{tot\_vol\_millbase} = \Sigma_{\text{all materials in millbase}} \text{mat\_wet\_vol(i)}$$

$$\text{tot\_wt\_millbase} = \Sigma_{\text{all materials in millbase}} \text{mat\_formula\_wt(i)}$$

$$\text{solids\_factor\_millbase} = \text{tot\_dry\_vol\_millbase} / \text{tot\_vol\_millbase}$$

$$\text{bead\_density\_factor} = \text{bead\_density}/2.5$$

$$\text{milling\_energy} = \text{milling\_power\_draw} * \text{milling\_time}$$

$$\text{specific\_milling\_energy} = \text{milling\_energy} / \text{tot\_wt\_millbase}$$

$$\text{milling\_efficiency} = \text{solids\_factor\_millbase} * \text{bead\_density\_factor} * \text{bead\_fill\_fraction} * \text{bead\_size}$$

$$\text{area\_to\_diameter\_factor\_millbase} = 0.006 * \text{solids\_factor\_millbase} / \text{tot\_wt\_millbase}$$

$$\text{surface\_area\_increase} = (\text{milling\_efficiency} * \text{specific\_milling\_energy}) / \text{tech\_surface\_energy}$$

$$\text{surface\_area\_50\_0\_millbase} = 1{,}000{,}000 * \text{area\_to\_diameter\_factor\_millbase} / \text{ps\_50\_initial}$$

$$\text{surface\_area\_50\_final\_millbase} = \text{surface\_area\_50\_0\_millbase} + \text{surface\_area\_increase}$$

Final particle sizes can be calculated from the intermediate properties as follows:

$$\text{ps\_50\_final} = 1{,}000{,}000 * \text{area\_to\_diameter\_factor\_millbase} / \text{surface\_area\_50\_final\_millbase}$$

$$ps\_90\_final = ps\_50\_final * d90\_to\_d50\_ratio$$

These relationships are shown in Fig. 2. Program code implementing this example follows from the teachings of the program code of Table 1. In this example, knowledge about material, process and equipment characteristics are interrelated. Material choice and level are represented by material_name and mat_formula_wt Process choice and parameters are given by mill_operation_type and and choices of milling time and bead_fill_fraction. Equipment choices and parameter are given by the mill_type and bead_type choices and the bead_size parameter.

Use of the above-described formulation knowledge web and databases will now be described. In the example that follows, a formulation knowledge web is used to determine processes and equipment that can result in desired target particle sizes for ps_50_final and PS_90_final.

Use of the millbase formulation knowledge web may begin with user-selection of a set of initial constraints. For this example, the user-selected constraints include the following material, target millbase, equipment choice, equipment parameter, process choice, and process parameter constraints:

| User-Specified Material Constraints: | | |
|---|---|---|
| **material_name** | **mat_formula_wt min (kg)** | **mat_formula_wt max (kg)** |
| Goal Tech | 49.95 | 49.95 |
| Kelzan | 0.05 | 00.05 |
| Proxel GXL | 0.05 | 00.05 |
| Water | 49.95 | 49.95 |

| User-Specified Target Millbase Constraints: | | |
|---|---|---|
| **Property** | **minimum (microns)** | **maximum (microns)** |
| ps_50_final | 0.9 | 1.1 |
| ps_90_final | | 3.5 |

| User-Specified Equipment Choice Constraints: | |
|---|---|
| **Equipment Type** | **Allowed Choices** |
| mill_type | Dynomill KD-15 (15 L) |
| bead_type | Glass - unleaded<br>Glass - leaded<br>Zircon Oxide (YTZ) |

| User-Specified Equipment Parameter Constraint: | | |
|---|---|---|
| **Property** | **minimum (mm)** | **maximum (mm)** |
| bead_size | 1 | 1 |

| User-Specified Process Choice Constraints | |
|---|---|
| **Process Type** | **Allowed Choices** |
| mill_operation_type | Continuous Recirculation<br>One Discrete Pass<br>Two Discrete Passes<br>Three Discrete Passes<br>Four or More Discrete Passes |

| User-Specified Process Parameter Constraints: | | |
|---|---|---|
| **Property** | **minimum** | **maximum** |
| milling_time (minutes) | 30 | 90 |
| bead_fill_fraction | 0.8 | 0.8 |

Given these constraints, the databases and the relationships the formulation engine can dynamically determine the processes needed to achieve the target particle sizes. Based on the described formulas and constraints, the formulation engine may reach the following conclusion with respect to mill operation type and operation time:

| mill_operation_type conclusion | |
|---|---|
| **mill_operation_type** | **result** |
| Continuous Recirculation | (No Solution) |
| One Discrete Pass | (No Solution) |
| Two Discrete Passes | Can Meet Constraints |
| Three Discrete Passes | Can Meet Constraints |
| Four or More Discrete Passes | Can Meet Constraints |

m

| milling time conclusion (range available before fixing mill_operation_tpe) | | |
|---|---|---|
| **Property** | **minimum (minutes)** | **maximum (minutes)** |
| milling_time | 79 | 90 |

For a particular bead type, the range of milling time will be dependent on the mill operation type. For example, if the bead type is constrained to be Zircon Oxide (YTZ), the range of available milling times will be as follows:

| **mill_operation_type selected** | **milling_time minimum (minutes)** | **milling_time maximum (minutes)** |
|---|---|---|
| Continuous Recirculation | (No Solution) | (No Solution) |
| One Discrete Pass | (No Solution) | (No Solution) |
| Two Discrete Passes | 88 | 90 |
| Three Discrete Passes | 81 | 90 |
| Four or More Discrete Passes | 79 | 90 |

Thus, one process choice for meeting the user-specified target constraints is to use Zircon Oxide (YTZ) beads in a mill that operates using three discrete passes for a milling time of 88 minutes. This yields a ps_50_final of 1.00 microns and a ps_90_final of 3.24 microns. Other choices within the solution space will also meet the user-specified constraints.

The invention may be implemented in digital electronic circuitry, or in computer hardware, firmware, software, or in combinations of them. Apparatus of the invention may be implemented in a computer program product tangibly embodied in a machine-readable storage device for execution by a programmable processor; and method steps of the invention may be performed by a programmable processor executing a program of instructions to perform functions of the invention by operating on input data and generating output. The invention may advantageously be implemented in one or more computer programs that are executable on a programmable system including at least one programmable processor coupled to receive data and instructions from, and to transmit data and instructions to, a data storage system, at least one input device, and at least one output device. Each computer program may be implemented in a high-level procedural or object-oriented programming language, or in assembly or machine language if desired; and in any case, the language may be a compiled or interpreted language. Suitable processors include, by way of example, both general and special purpose microprocessors. Generally, a processor will receive instructions and data from a read-only memory and/or a random access memory. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM disks. Any of the foregoing may be supplemented by, or incorporated in, specially-designed ASICs (application-specific integrated circuits).

A number of embodiments of the present invention have been described. Nevertheless, it will be understood that various modifications may be made without departing from the spirit and scope of the invention. For example, other programming languages may be used to implement a formulation knowledge engine. The formulation knowledge engine may be used with a graphical user-based interface or a web-based interface. Material, process, and other data items may be stored in an external database, such as structured query language (SQL) - based database, a spreadsheet, or other data storage system and accessed by program code implementing the formulation engine using standard application programming interfaces (APIs).

The formulation invention described herein may be used to address formulation problems in a variety of industries. Industries that may benefit from the invention include food preparation, building, petroleum, cosmetics, electronics, plastics, material manufacturing, and environmental industries. In the food preparation industry, a formulation engine can be implemented to reformulate combining, cooking, and processing food ingredients as well as to reformulate processes such as fermenting and blending of wine or beer. In the building industry, processes used to produce building products, such as the compounding of cement, sand, and other solid materials with water to produce mortar can be reformulated. In the petroleum industry, chemical blends (e.g., processing and mixing basic oils and additives to prepare motor oil may be reformulated). In the plastics industry, formulation of a molded polymer from polymer beads and plastics additive materials (impact modifiers, pigments, etc). In the environmental management industry, water treatment using resins, settling, and other processes can be reformulated. In general all of these examples involve formulation of one or more materials or composite materials from one or more ingredient materials, along with information on the process steps, conditions and equipment used in the preparation. In the claims that follow, these ingredients and processes are referred to as formulation components. Accordingly, other embodiments are within the scope of the following claims.

## Claims

1.    A computer implemented method of formulating a product, the method comprising:

receiving a product formulation script, the script comprising:

a component selection option, the selection option comprising a plurality of sets of formulation components,
a formulation component constraint identifying a range associated with a formulation component property, and
a target constraint; and

processing the product formulation script to determine a collection of formulation components that can be used to produce a product, the product having a first property in accordance with the target constraint, wherein the collection comprises the first formulation component and processing the script comprises:

determining a first one of the sets of formulation components for inclusion in the collection; and
determining a permitted range associated with the first formulation component in the collection, the permitted range being compliant with the formulation component constraint.

2.    The method of claim 1 wherein processing the script comprises processing based on a formulation knowledge web, the formulation knowledge web interrelating properties of the formulation components to target properties.

3.    The method of claim 2 wherein processing the script further comprises retrieving formulation component properties from a database.

4.    The method of claim 3 wherein the formulation component properties retrieved from the database comprise a property of a formulation component comprising a raw material and a property of a formulation component comprising a manufacturing process.

5. The method of claim 1 wherein processing the script further comprises excluding at least one of the other sets of formulation components from the collection.

6. The method of claim 1 wherein the first formulation component comprises a formulation component in the first set.

7. The method of claim 1 wherein the first formulation component comprises a formulation component not in the first set.

8. The method of claim 1 wherein the range identified by the formulation constraint comprises the permitted range and an impermissible range and determining the permitted range comprises determining a range of values enabling formulation of the product in accordance with the target constraint.

9. The method of claim 1 wherein:
   the target constraint comprises a range of values comprising attainable values of the first property and unattainable values of the first property, and processing the script comprises determining the attainable values and the unattainable values.

10. A computer system for formulating a chemical product, the system comprising:

   a processor coupled to a user input device;
   a database coupled to the processor and comprising instructions to configure the processor to:

   receive a product formulation script, the script comprising:

   a component selection option, the selection option comprising a plurality of sets of formulation components,
   a formulation component constraint identifying a range associated with a formulation component property, and
   a target constraint; and

   process the product formulation script to determine a collection of formulation components that can be used to produce a product, the product having a first property in accordance with the target constraint, wherein the collection comprises the first formulation component and the instructions to process the script comprise instructions to:

   determine a first one of the sets of formulation components for inclusion in the collection; and
   determine a permitted range associated with the first formulation component in the collection, the permitted range being compliant with the formulation component constraint.

FIG. 1A

FIG. 1B

Millbase Property Targets

ps_90_final

ps_50_final

surface_area_50_final_millbase

surface_area_increase

specific_milling_energy

surface_area_50_0_millbase

milling_efficency

area_to_diameter_factor

solids_factor_millbase

FIG. 2A

| tot_dry_vol_millbase | tot_vol_millbase | tot_wt_millbase |
|---|---|---|

| mat_dry_vol | mat_wet_vol |
|---|---|

| mat_dry_wt | | bead_density_factor | miiling_energy |
|---|---|---|---|

**Formulation Parameters**

material_name
mat_formula_wt
mill_type
bead_type
bead_size
mill_operation_type
milling_time
bead_fill_fraction

**Material Database**

material_name
mat_wt_solids
mat_dry_density
mat_supplied_density
tech_surface_energy
ps_50_initial

**Process Database**

mill_operation_type
d90_to_d50_ratio

**Equipment Database**

mill_type
milling_power_draw
bead_type
bead_density

FIG. 2B